(19) Europäisches Patentamt
European Patent Office
Office européen des brevets

(11) Veröffentlichungsnummer: **0 339 555 B1**

(12) **EUROPÄISCHE PATENTSCHRIFT**

(45) Veröffentlichungstag der Patentschrift: **23.12.92**

(51) Int. Cl.⁵: **A61K 31/53**, A61K 9/08, A61K 47/22

(21) Anmeldenummer: **89107413.0**

(22) Anmeldetag: **25.04.89**

# (54) Wasserlösliche Zubereitungen von Coccidiostatica.

(30) Priorität: **28.04.88 DE 3814323**

(43) Veröffentlichungstag der Anmeldung:
**02.11.89 Patentblatt 89/44**

(45) Bekanntmachung des Hinweises auf die Patenterteilung:
**23.12.92 Patentblatt 92/52**

(84) Benannte Vertragsstaaten:
**AT BE CH DE ES FR GB IT LI NL**

(56) Entgegenhaltungen:
**EP-A- 0 116 175**
**EP-A- 0 154 885**
**EP-A- 0 179 583**
**EP-A- 0 215 354**
**US-A- 3 361 627**

(73) Patentinhaber: **HOECHST AKTIENGESELLSCHAFT**
**Postfach 80 03 20**
**W-6230 Frankfurt am Main 80(DE)**

(72) Erfinder: **Hiller, Dietrich, Dr.**
**Riederbergstrasse 11**
**W-6200 Wiesbaden(DE)**
Erfinder: **Hornykiewytsch, Theopil, Dr.**
**Johannesallee 28**
**W-6230 Frankfurt am Main 80(DE)**
Erfinder: **Raether, Wolfgang, Dr.**
**Falkensteinstrasse 6**
**W-6072 Dreieich(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft flüssige Zubereitungen von Coccidiostatica, die mit dem Trinkwasser an Tiere verabreicht werden können.

N-Phenyltriazine der allgemeinen Formeln I und II

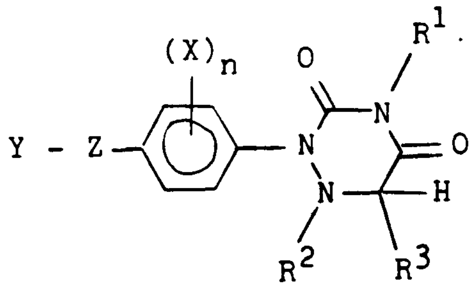

(I)

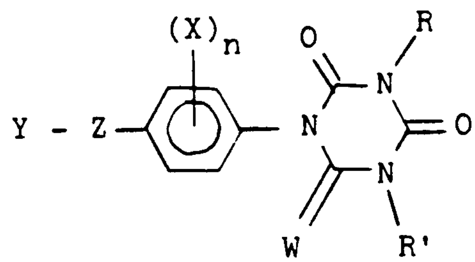

(II)

in denen

| | |
|---|---|
| $R^1$, R und R' | unabhängig voneinander Wasserstoff oder $C_1$-$C_4$-Alkyl, |
| $R^2$ | zusammen mit $R^3$ eine Doppelbindung oder unabhängig davon Wasserstoff oder $C_1$-$C_4$-Alkyl, |
| $R^3$ | zusammen mit $R^2$ eine Doppelbindung oder unabhängig davon Wasserstoff oder $C_1$-$C_4$-Alkyl, |
| W | Sauerstoff oder Schwefel, |
| X | jeweils unabhängig voneinander Halogen, wie Fluor, Chlor und Brom, $C_1$-$C_4$-Alkyl, Trifluormethyl, Cyano, Thiocyanato, $C_1$-$C_4$-Alkylthio, Nitro oder $C_1$-$C_4$-Alkoxy, |
| n | 0, 1, 2, 3 oder 4, vorzugsweise 0 bis 2, |
| Y | Wasserstoff, $C_1$-$C_6$-Alkyl, $C_1$-$C_6$-Halogenoalkyl, Benzyl, Phenyl oder Phenyl, das durch mindestens einen Rest aus der Gruppe Alkyl, Alkoxy, Halogenoalkyl, Halogenoalkoxy, Halogen, Alkylthio, Alkylsulfinyl, Alkylsulfonyl, Halogenalkylthio, Halogenalkylsulfinyl, und Halogenalkylsulfonyl mit jeweils 1 bis 4 C-Atomen im Alkylrest substituiert ist, Thienyl oder Halothienyl und |
| Z | eine direkte Bindung oder eine divalente Gruppe der Formel O, S, SO, $SO_2$, NH, $NR^0$, worin $R^0$ Wasserstoff oder $C_1$-$C_4$-Alkyl ist, oder $C(CN)(R^+)$, worin $R^+$ Wasserstoff oder Methyl ist, |

bedeuten,

sind als Wirkstoffe gegen Coccidiose und ähnliche Erkrankungen bei Tieren bekannt; vergleiche bspw. die europäischen Patentanmeldungen mit den Veröffentlichungs-Nummern 0 215 354, 0 154 885 (US-A-4,640,917), 0 170 316 und 0 232 932 sowie die deutschen Offenlegungsschriften Nrs. 2 413 722, 2 718 799 und 2 722 537 (US-A-4,198,407) sowie die US-Patentschrift Nr. 3,905,971.

Zur Behandlung der Coccidiose mußten diese Wirkstoffe bisher in fester, kristalliner Form dem Futter der Tiere zugesetzt werden. Für eine Behandlung über das Trinkwasser konnte bisher keine generell geeignete Anwendungsform gefunden werden. Dagegen entspricht gerade die Behandlung über das Trinkwasser, sofern dies möglich ist, oft in idealer Weise den Bedürfnissen des Tierhalters, insbesondere in der Nutztierhaltung. So kommt es beispielsweise häufig vor, daß an Coccidiose erkrankte Tiere die Futteraufnahme verweigern, während die Bereitschaft zur Aufnahme von Trinkwasser noch vorhanden ist.

Eine Therapie der Coccidiose über das wirkstoffhaltige Futter ist daher häufig erschwert oder unmöglich.

Um eine sichere Anwendung über das Trinkwasser zu gewährleisten, muß der Wirkstoff im Wasser in stabiler und wirksamer Form homogen verteilt vorliegen. Die Wirkstoffe der obengenannten Formeln (I) und (II) sind in Wasser nicht löslich. Aus einer in der Anwendungskonzentration hergestellten Suspension sedimentieren sie noch während des Anwendungszeitraums ganz oder teilweise, wobei die Anwendungskonzentration je nach Tierart und Schwere der Erkrankung in der Regel bei 1 bis 500 ppm, vorzugsweise meisten bei 2 bis 100 ppm, insbesondere bei 2 bis 30 ppm, liegt. Der Anwendungszeitraum hängt von den Gegebenenheiten der Fütterung der Tiere ab. Das mit dem Wirkstoff versetzte Trinkwasser sollte jedoch mindestens 24 Stunden, möglichst länger als 24 Stunden, als stabile wäßrige Lösung oder Suspension vorliegen. Für die Coccidiostatica der Formel (I) und (II) läßt sich eine über diesen Zeitraum stabile wäßrige Anwendungsform mit definiertem Wirkstoffgehalt ohne den Einsatz von Hilfsstoffen somit nicht herstellen.

Die Wirkstoffe der Formeln (I) und (II) lassen sich in verschiedenen organischen Lösemitteln gut lösen, bspw. in Aceton, Alkoholen, Dimethylsulfoxid, Essigsäureethylester oder N-Methylpyrrolidon. Beim Verdünnen dieser Lösungen mit Wasser auf die Anwendungskonzentration fallen die Wirkstoffe aber sofort oder nach kurzer Zeit wieder aus.

Aus der DE-A-3 300 793 sind wassermischbare Lösungen des Wirkstoffs der Formel (II), in der R Wasserstoff, R' Methyl, n = 1, X = 3-Methyl, W = O und Y-Z = 4-Trifluormethylthiophenoxy bedeuten, bekannt. Diese Wirkstofflösungen, die ein oder mehrere polare Lösemittel und alkalisch reagierende Stoffe enthalten, können mit Wasser auf die Anwendungskonzentration der Wirkstoffe verdünnt werden, ohne daß der Wirkstoff innerhalb von 24 Stunden ausfällt.

Diese Methode zur Herstellung stabiler wäßriger Wirkstofflösungen läßt sich nicht auf Wirkstoffe der allgemeinen Formel (I) übertragen.

Aus der DE-A-3 300 793 ist ferner bekannt, daß eine Lösungsvermittlung im wäßrigen Medium durch Zusatz von Solubilisatoren, wie bspw. polyoxyethyliertem Rizinusöl oder Polyoxyethylen-Sorbitan-Fettsäureestern, bei dem genannten Wirkstoff der Formel (II) nicht zum Erfolg führt. Das Ausfallen des Wirkstoffs wird zwar um einige Stunden verzögert, nicht jedoch über einen Zeitraum von 24 Stunden.

Auch Wirkstoffe der allgemeinen Formel (I) lassen sich in Wasser, das Solubilisatoren in üblicher, d.h. in für die Anwendung nicht störender Konzentration (0,5 bis 5 Gew.-%) enthält, in der benötigten Anwendungskonzentration nicht lösen.

Es besteht somit ein Bedarf nach Zubereitungen von Coccidiostatica, die mit Trinkwasser auf die Anwendungskonzentration der Wirkstoffe verdünnt werden können, ohne daß die Wirkstoffe innerhalb kurzer Zeit ausfallen.

Gegenstand der Erfindung sind Zubereitungen mit einem oder mehreren Wirkstoffen der obengenannten Formeln (I) und (II), gekennzeichnet durch

a) bis zu 10000 ppm, bezogen auf das Gewicht der Zubereitung, an Wirkstoffen,

b) ein mit Wasser mischbares, physiologisch unbedenkliches, anionisches oder nichtionisches Tensid oder ein Gemisch mehrerer dieser Tenside und

c) 0 bis 80 Gew.-%, bezogen auf die Zubereitung, eines physiologisch unbedenklichen, wasserlöslichen organischen Lösemittels

d) 0 bis 70 Gew.-%, bezogen auf die Zubereitung, an Wasser.

Im allgemeinen liegt der pH-Wert einer erfindungsgemäßen Zubereitung - insofern er bei Anwesenheit von restlichem Wasser gemessen werden kann - in einem Bereich von weniger als pH 8. Nach Verdünnung mit dem Trinkwasser auf die Anwendungskonzentration soll das Trinkwasser praktisch neutral oder leicht sauer reagieren, vorzugsweise pH 6 bis 7 aufweisen.

Beim Verdünnen der erfindungsgemäßen Zubereitungen mit Trinkwasser auf die Anwendungskonzentration der Coccidiostatica von 1 bis 500 ppm, vorzugsweise 2 bis 100 ppm, insbesondere 2 bis 30 ppm, bleiben die erhaltenen wäßrigen Wirkstofflösungen über längere Zeit stabil, d.h. bis zu mehreren Tagen und mindestens länger als 24 Stunden. Die Stabilität der Lösungen ist überraschend, weil der Anteil der Solubilisatoren im Trinkwasser in der Regel sogar wesentlich unterhalb der obengenannten üblichen Konzentration von 0,5 bis 5 Gew-% liegt, bei dessen Vorliegen im Trinkwasser die Wirkstoffe nachträglich nicht mehr unter Bildung einer stabilen wäßrigen Lösung gelöst werden können. Vorzugsweise ist die Konzentration des Solubilisators im Trinkwasser, d.h. nach dem Verdünnen der erfindungsgemäßen Wirkstoffzubereitungen, bei 0,001 bis 0,1 Gew.-%, insbesondere 0,001 bis 0,01 Gew.-% Solubilisator.

Durch die erfindungsgemäßen, mit Wasser mischbaren Lösungskonzentrate (Zubereitungen) wurde eine technisch leicht realisierbare und wirtschaftliche Möglichkeit gefunden, stabile wäßrige Lösungen von sonst in Wasser nicht in der Anwendungskonzentration löslichen Wirkstoffen herzustellen.

Von besonderem Interesse sind erfindungsgemäße Zubereitungen, die einen Wirkstoff der obengenannten Formel (I) enthalten, in der

$R^1$ Wasserstoff oder $C_1$-$C_4$-Alkyl, vorzugsweise Wasserstoff oder Methyl,
$R^2$ zusammen mit $R^3$ eine Doppelbindung oder
$R^2$ und $R^3$ unabhängig voneinander Wasserstoff oder $C_1$-$C_4$-Alkyl, insbesondere Wasserstoff oder Methyl,
X jeweils unabhängig voneinander Chlor, Brom, $C_1$-$C_4$-Alkyl, vorzugsweise Methyl, Trifluormethyl oder
$C_1$-$C_4$-Alkoxy,
n 0, 1 oder 2,
Y Wasserstoff, $C_1$-$C_4$-Halogenoalkyl, insbesondere $C_1$-$C_3$-Alkyl mit ein oder mehreren Fluoratomen als Substituenten, Phenyl oder Phenyl, das durch mindestens einen Rest aus der Gruppe Methyl, Methoxy, Trifluormethyl, Chlor, Methylthio, Methylsulfinyl, Methylsulfonyl, Trifluormethylthio, Trifluormethylsulfinyl oder Trifluormethylsulfonyl substituiert ist, und
Z eine direkte Bindung oder Sauerstoff bedeuten.

Bevorzugt sind erfindungsgemäße Zubereitungen mit einem Wirkstoff a) der Formel (Ia)

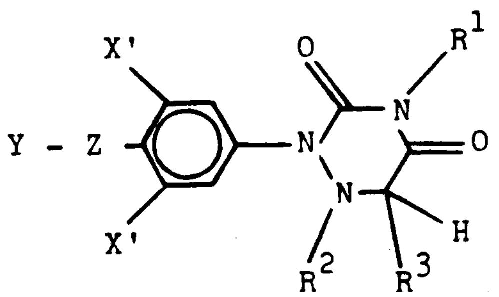

(Ia)

in der
$R^1$ Wasserstoff oder Methyl,
$R^2$ zusammen mit $R^3$ eine Doppelbindung oder
$R^2$ und $R^3$ unabhängig voneinander Wasserstoff oder Methyl,
X' Wasserstoff, Chlor oder Trifluormethyl und
Y-Z- Wasserstoff, $C_1$-$C_4$-Halogenalkoxy, insbesondere Tetrafluorethoxy oder Hexafluorpropoxy, oder $C_1$-$C_4$-Alkylsulfonyl-phenoxy, insbesondere 4-Methylsulfonyl-phenoxy,
bedeuten.

Von besonderem Interesse sind auch erfindungsgemäße Zubereitungen mit einem Wirkstoff a) der Formel (II), in der
R und R' unabhängig voneinander Wasserstoff oder Methyl,
W Sauerstoff,
X jeweils unabhängig voneinander Chlor, $C_1$-$C_4$-Alkoxy, $C_1$-$C_4$-Alkyl, oder Trifluormethyl,
n 0, 1 oder 2,
Y Phenyl oder Phenyl, das durch mindestens einen Substituenten aus der Gruppe Chlor, Trifluormethyl, Trifluormethoxy, Trifluormethylthio, Trifluormethylsulfinyl oder Trifluormethylsulfonyl substituiert ist, und
Z Sauerstoff bedeuten.

Bevorzugte Zubereitungen mit einem Wirkstoff a) der Formel (II) sind solche, in denen in Formel (II) $R^1$ Wasserstoff, $R^2$ Methyl, W Sauerstoff, X 3-Methyl, n 1, und die Gruppe Y-Z- den Rest 4-Trifluormethylthiophenoxy bedeuten.

Als Hilfsstoffe b) der erfindungsgemäßen Zubereitung kommen anionische und nichtionische Tenside in Betracht, die mit Wasser mischbar sind, in denen der Wirkstoff in genügender Konzentration löslich ist und die physiologisch unbedenklich sind. Geeignete nichtionische Tenside gehören beispielsweise zu der Gruppe der Ether bzw. Ester von Oligoethylenglykolethern mit Verbindungen aus der Gruppe der $C_{12}$-$C_{18}$-Fettalkohole, $C_{12}$-$C_{18}$-Fettsäuren, $C_{12}$-$C_{18}$-Hydroxyfettsäuren, $C_{12}$-$C_{18}$-Hydroxyfettsäureester, Zuckeralkohole, Zuckerfettsäureester und anderen Polyalkohole, wie Glycerin, Glycerinfettsäureester, Sorbitanfettsäureester oder Glycerin-Sorbitanfettsäureester. Beispiele für derartige nichtionische Tenside sind POE-Sorbitanmonolaurat, POE-Sorbit, POE-Rizinusöl, POE-hydriertes Rizinusöl, POE-Laurylalkohol, -Myristylalkohol, -Cetylalkohol, -Stearylalkohol und -Oleylalkohol, POE-Glycerolsorbitanfettsäureester, POE-Fettsäureester mit Fettsäuren wie Laurylsäure, Myristylsäure, Palmitinsäure, Stearinsäure oder Ölsäure, POE-Triglyceride,

wobei jeweils POE eine bzw. mehrere Polyoxyethylen-Ketten bedeutet, die in der Regel durch Oxalkylierung der bezeichneten Grundkörper gebildet werden.

Bevorzugte nichtionische Tenside haben einen HLB-Wert (hydrophilic-lipophilic balance) von mehr als 14. Bevorzugte nichtionische Tenside sind POE-Sorbitanfettsäureester, bspw. POE-(20)Sorbitanmonolaurat (die Zahl in der Klammer bedeutet hier und im folgenden jeweils die Gesamtzahl der im Tensidmolekül enthaltenen Ethylenoxy-Einheiten), POE-(20)Sorbitanmonostearat, POE-(20)Sorbitanmonooleat, POE-(20)-Sorbitanmonopalmitat, sowie Fettalkoholoxethylate, wie z.B. die Ether POE-(12)Laurylalkohol, POE-(20)-Stearylalkohol, POE-(20)Oleylalkohol, POE-(20)Cetylalkohol, POE-(23)Laurylalkohol, sowie Fettsäureoxethylate, wie der Ester POE-(40)Stearinsäure oder POE-(50)Stearinsäure. Beispiele für anionische Tenside sind Fettalkoholsulfate, Fettsäuresalze und Fettalkoholethersulfate, vorzugsweise Fettalkoholethersulfate wie Natrium-laurylethersulfat.

Die erfindungsgemäßen Zubereitungen können bis zu 80 Gew.-%, bezogen auf die Zubereitung, eines physiologisch unbedenklichen, wasserlöslichen organischen Lösungsmittels enthalten.

Geeignete Lösungsmittel sind beispielsweise niedere aliphatische Alkohole, wie Ethylalkohol und Isopropylalkohol, mehrwertige Alkohole, wie Glycerin, Propylenglykol, Polyethylenglykole, Blockpolymere von Ethylenoxid und Propylenoxid, sowie Arylalkanole, wie Benzylalkohol. Außerdem sind geeignet Ketone, bspw. Aceton und Methylethylketon, oder Ester, wie Milchsäureethylester.

Im Gegensatz zu den wassermischbaren Lösungen aus der DE-A-33 00 793 ist es nicht erforderlich, den erfindungsgemäßen Zubereitungen basische Stoffe zuzusetzen.

Die erfindungsgemäßen Zubereitungen können auch andere Hilfsstoffe, wie Konservierungsmittel, Antioxidantien, weitere Suspensionsstabilisatoren, Verdickungsmittel, wie Methylcellulose und kolloidale Kieselsäure, sowie Aufbaustoffe zur Tierernährung, Farb- und Aromastoffe sowie physiologisch verträgliche Säuren oder Puffersubstanzen enthalten. Der Anteil der genannten Hilfsstoffe soll in der Regel nicht mehr als 20 Gew.-%, vorzugsweise nicht mehr als 10 Gew.-% der Zubereitung ausmachen.

Die Zubereitungen können schon bei der Herstellung, d.h. vor dem Verdünnen mit Wasser bzw. dem Trinkwasser schon einen Wassergehalt aufweisen. Die Höhe dieses Wassergehalts hängt im allgemeinen von dem verwendeten Solubilisator und dem eventuell eingesetzten organischen Lösungsmittel ab. In der Regel sind jedoch Wassergehalte von weniger als 10 Gew.-%, bezogen auf das Gewicht der Zubereitung, bevorzugt.

Bevorzugte erfindungsgemäße Zubereitungen bestehen aus

a) 100 bis 8000 ppm, insbesondere 2000 bis 6000 ppm, bezogen auf die Zubereitung, an einem Wirkstoff der Formel (I) oder (II),

b) einem anionischen oder nichtionischen Tensid mit einem HLB-Wert von mehr als 14,

c) 0 bis 60 Gew.-%, insbesondere 0 Gew.-%, bezogen auf die Zubereitung, eines physiologisch unbedenklichen wasserlöslichen organischen Lösungsmittels,

d) 0 bis 10 Gew.-%, insbesondere 0 bis 5 Gew.-%, bezogen auf die Zubereitung, an Wasser.

Gegenstand der Erfindung ist auch das Verfahren zur Herstellung einer erfindungsgemäßen Zubereitung, dadurch gekennzeichnet, daß man einen oder mehrere Wirkstoffe der obengenannten Formeln (I) und (II) in einem mit Wasser mischbaren, physiologisch unbedenklichen anionischen oder nichtionischen Tensid oder einem Gemisch mehrerer dieser Tenside und gegebenenfalls in Gegenwart von bis zu 80 Gew.-%, bezogen auf die Zubereitung, eines physiologisch unbedenklichen, wasserlöslichen organischen Lösungsmittels und gegebenenfalls in Gegenwart von bis zu 70 Gew.-%, bezogen auf die Zubereitung, an Wasser löst, wobei die entstandene Zubereitung bis zu 10000 ppm Wirkstoffgehalt aufweist.

Die Herstellung der erfindungsgemäßen Zubereitungen kann beispielsweise so durchgeführt werden, daß man alle Bestandteile der Zubereitung in ein Gefäß einwiegt und dann unter Erwärmen solange rührt, bis eine klare Lösung entstanden ist. Auch ist es beispielsweise möglich, die Mischung der Bestandteile einige Zeit gegebenenfalls bei erhöhter Temperatur zu rühren, die Mischung dann zu filtrieren und das Filtrat weiterzuverwenden, sofern ohne Filtration keine klare Lösung entstanden ist.

Gegenstand der Erfindung ist auch die Verwendung der erfindungsgemäßen Zubereitung zur Herstellung von wirkstoffhaltigen (trinkfertiger wäßriger lösungen) zur Behandlung und Prophylaxe der Coccidiose bei Tieren, dadurch gekennzeichnet, daß die erfindungsgemäßen Zubereitungen dem Trinkwasser zugesetzt werden.

Die Menge an Zubereitung, die dem Trinkwasser zugesetzt wird, hängt von der Wirksamkeit des einzelnen verwendeten Coccidiostatikums, der Tierart und der Schwere der Erkrankung ab. In der Regel wird soviel Zubereitung dem Trinkwasser zugesetzt, daß eine Konzentration des Wirkstoffs von 1 bis 500 ppm, vorzugsweise 2 bis 100 ppm, insbesondere 2 bis 30 ppm, im Trinkwasser vorliegt.

Es ist zweckmäßig, die erfindungsgemäßen Zubereitungen dem Trinkwasser erst relativ kurze Zeit vor dessen Verwendung zuzusetzen. Die erfindungsgemäßen Wirkstoffzubereitungen sind in der Regel mehrere

Monate lagerstabil. Die aus den konzentrierten Zubereitungen hergestellten verdünnten Lösungen zur Verabreichung als Trinkwasser sind in der Regel mehrere Tage stabil, mindestens jedoch für 24 Stunden.

In Tierversuchen wurde die gute Verträglichkeit und volle Wirksamkeit der unter Verwendung erfindungsgemäßer Wirkstoffzubereitungen hergestellten Trinkwässer nachgewiesen.

## Herstellungsbeispiele

Unter Verwendung der in der folgenden Tabellen 1a) und 1b) angegebenen Wirkstoffe der Formeln (I) und (II) werden unter Verwendung der in der Tabelle 2 angegebenen Hilfsstoffe und Mengenverhältnisse, die jeweiligen Wirkstoffe unter Erwärmen gelöst.

**Tabellen 1 a) und 1 b)**

a)

| Nr. | Formel | $R^1$ | $R^2$ | $R^3$ | X' | Y-Z- |
|---|---|---|---|---|---|---|
| W 1 | Ia | H | - | - | Cl | $CF_2HCF_2$-O |
| W 2 | Ia | H | - | - | Cl | $CF_3$-CHF-$CF_2$-O |
| W 3 | Ia | H | H | H | Cl | 4-($CH_3SO_2$)-$C_6H_4$O- |
| W 4 | Ia | H | $CH_3$ | H | Cl | 4-($CH_3SO_2$)-$C_6H_4$O- |
| W 5 | Ia | $CH_3$ | - | - | $CF_3$ | H |
| W 6 | Ia | $CH_3$ | H | H | $CF_3$ | H |
| W 7 | Ia | $CH_3$ | - | - | H | $CF_3$-CHF-$CF_2$-O- |

b)

| Nr. | Formel | W | R | R' | YZ | n | X |
|---|---|---|---|---|---|---|---|
| W 8 | II | O | H | $CH_3$ | 4-$CF_3$S-$C_6H_4$O | 1 | 3-$CH_3$ |

Tabelle 2

| Bsp. | Wirkstoff | Tensid |
|---|---|---|
| 1 | 4 Teile W 1 | 1000 Teile POE-(20)-Sorbitanmonolaurat |
| 2 | 1 Teil W 1 | 100 Teile " |
| 3 | 1 Teil W 1 | 1000 Teile " |
| 4 | 4 Teile W 2 | 1000 Teile " |
| 5 | 4 Teile W 3 | 1000 Teile " |
| 6 | 4 Teile W 4 | 1000 Teile " |
| 7 | 4 Teile W 5 | 1000 Teile " |
| 8 | 4 Teile W 6 | 1000 Teile " |
| 9 | 4 Teile W 7 | 1000 Teile " |
| 10 | 4 Teile W 8 | 1000 Teile " |
| 11 | 1 Teil W 8 | 100 Teile " |
| 12 | 1 Teil W 8 | 1000 Teile " |
| 13 | 1 Teil W 3 | 1000 Teile " |
| 14 | 6 Teile W 1 | 1000 Teile " |
| 15 | 4 Teile W 1 | 1000 Teile Natriumlaurylethersulfat |
| 16 | 4 Teile W 3 | 1000 Teile " |
| 17 | 4 Teile W 8 | 1000 Teile " |
| 18 | 4 Teile W 1 | 1000 Teile einer 1:1-Mischung von POE-(20)-Sorbitanmonolaurat und Natriumlaurylethersulfat |
| 19 | 4 Teile W 1 | 1000 Teile einer 1:1-Mischung von Natriumlaurylethersulfat und POE-(20)-Sorbitanmonolaurat |
| 20 | 4 Teile W 1 | 1000 Teile einer 2:8-Mischung von POE-(20)-Sorbitanmonolaurat und Ethanol |
| 21 | 4 Teile W 5 | 1000 Teile POE-(23)-Laurylalkohol |
| 22 | 4 Teile W 5 | 1000 Teile POE-(20)-Oleylalkohol |
| 23 | 4 Teile W 5 | 1000 Teile POE-(20)-Sorbitanmonooleat |

**Theraphiebeispiele**

Zur Behandlung von Coccidiose bei Tieren wurden die Zubereitungen 1 bis 23 (Tabelle 2) mit Wasser auf die Anwendungskonzentration verdünnt und den Tieren ad libitum verabreicht.

Therapieversuche wurden beispielsweise mit einem Mischisolat von Eimeria tenella und Eimeria acervulina (Tab. 3) und mit einem sensitiven Stamm von Eimeria tenella (Tab. 4) durchgeführt. In Batterien gehaltene 3 Tage alte Lohmann selected Leghorn-Hähnchen erhielten vom D + 2 (2 Tage nach Infektion) bis D + 3 medikiertes Trinkwasser ad libitum. Am DO (Tag der Infektion) wurden die medikierten Gruppen (12 Tiere/Gruppe) und die Infektionskontrolle infiziert, und zwar jedes Tier mit 1 x $10^5$ versporten Oocysten. Eine Gruppe diente als nicht infizierte, nicht medikierte Kontrolle. Handelsübliches Kükenaufzuchtfutter wurde zur freien Aufnahme angeboten.

Für die Beurteilung des coccidiostatischen oder coccidioziden Effektes wurden folgende Parameter untersucht (vgl. J. Johnson, W.M. Reid, Anticoccidial Drugs: Lesion Scoring Techniques in Battery and Floor-Pen Experiments with Chickens, Exp. Parasitol. 28, 30-36 (1970)):

Kotbefunde am D + 4, D + 6;
Feststellung der Mortalität infolge Coccidiose;
Gewichtszunahme: D0 bis D + 7;
pathologisch-anatomische Veränderung im Darmtrakt (lesion scores: 0-4) zwischen D + 4 und D + 7.

Die Ergebnisse sind in Tabellen 3 und 4 zusammengestellt.

Vor allem aus der geringeren mittleren Zahl an pathologischanatomischen Veränderungen (lesion scores) im Darmtrakt der Tiere im Vergleich zur infizierten Kontrolle wird die wirksame Therapie erkennbar. Analoge Ergebnisse werden mit den übrigen Zubereitungen aus Tabelle 2 erhalten.

**Tabelle 3** Therapieversuch (Küken) mit einem Mischisolat von E. tenella und E. acervulina

| Zubereitung Nr. | WIRKSTOFF-GEHALT mg/l | KOTBEFUNDE D+4 | KOTBEFUNDE D+6 | MORTALITÄT COCC. Tiere/Gesamt | GEWICHTSZUNAHME D0 bis D+7 Gramm | GEWICHTSZUNAHME D0 bis D+7 % | LESION SCORES (Durchschnittswerte) D+4 bis D+7 Caecum | LESION SCORES (Durchschnittswerte) D+4 bis D+7 Duodenum |
|---|---|---|---|---|---|---|---|---|
| 6 | 5 | 0 | 0-1 | 0 / 12 | 28,1 | 106,4 | 0,1 | 0,3 |
| | 2,5 | 0 | 0-1 | 0 / 12 | 25,7 | 97,3 | 0,2 | 1,1 |
| nicht medikierte, infizierte Kontrolle | 0 | 2 | 3 | 2 / 12 | 9,1 | 34,5 | 1,9 | 2,0 |
| nicht medikierte, nicht infizierte Kontrolle | 0 | 0 | 0 | 0 / 12 | 26,4 | 100 | 0 | 0 |

**Tabelle 4** Therapieversuch (Küken) mit einem Stamm Eimeria tenella

| Zubereitung Nr. | Wirkstoff-gehalt mg/l | Kotbefunde D+4 | Kotbefunde D+6 | Mortalität COCC. Tiere/Gesamt | Gewichtszunahme D0 bis D+7 Gramm | Gewichtszunahme D0 bis D+7 % | Lesion Scores (Durchschnittswerte) D+4 bis D+7 Caecum |
|---|---|---|---|---|---|---|---|
| 4 | 10 | 0 | 0 | 0 / 12 | 37,1 | 94,6 | 0,4 |
| nicht medikierte, infizierte Kontrolle | 0 | 2-3 | 2 | 4 / 12 | 20,9 | 53,3 | 2,3 |
| nicht medikierte, nicht infizierte Kontrolle | 0 | 0 | 0 | 0 / 12 | 39,2 | 100 | 0 |

**Patentansprüche**

**Patentansprüche für folgende Vertragsstaaten : AT, BE, CH, DE, FR, GB, IT, LI, NL**

**1.** Wirkstoffzubereitung gekennzeichnet durch einen Gehalt von

a) bis zu 10000 ppm, bezogen auf das Gewicht der Zubereitung, an einem oder mehreren

Coccidiostatika der allgemeinen Formeln I oder II

(I)

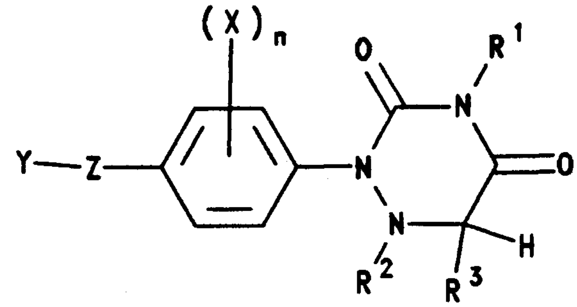

(II)

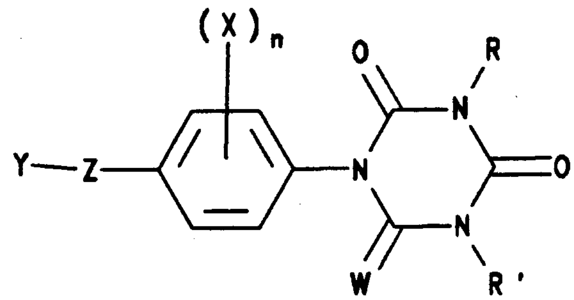

in denen

| | |
|---|---|
| $R^1$, R und R' | unabhängig voneinander Wasserstoff oder $C_1$-$C_4$-Alkyl, |
| $R^2$ | zusammen mit $R^3$ eine Doppelbindung oder unabhängig davon Wasserstoff oder $C_1$-$C_4$-Alkyl, |
| $R^3$ | zusammen mit $R^2$ eine Doppelbindung oder unabhängig davon Wasserstoff oder $C_1$-$C_4$-Alkyl, |
| W | Sauerstoff oder Schwefel, |
| X | jeweils unabhängig voneinander Halogen, wie Fluor, Chlor und Brom, $C_1$-$C_4$-Alkyl, Trifluormethyl, Cyano, Thiocyanato, $C_1$-$C_4$-Alkylthio, Nitro oder $C_1$-$C_4$-Alkoxy, |
| n | 0, 1, 2, 3 oder 4, vorzugsweise 0 bis 2, |
| Y | Wasserstoff, $C_1$-$C_6$-Alkyl, $C_1$-$C_6$-Halogenalkyl, Benzyl, Phenyl oder Phenyl, das durch mindestens einen Rest aus der Gruppe Alkyl, Alkoxy, Halogenoalkyl, Halogenoalkoxy, Halogen, Alkylthio, Alkylsulfinyl, Alkylsulfonyl, Halogenalkylthio, Halogenalkylsulfinyl und Halogenalkylsulfonyl mit jeweils 1 bis 4 C-Atomen im Alkylrest substituiert ist, Thienyl oder Halothienyl und |
| Z | eine direkte Bindung oder eine divalente Gruppe der Formel O, S, SO, $SO_2$, $NR^0$, worin $R^0$ Wasserstoff oder $C_1$-$C_4$-Alkyl ist, oder $C(CN)(R^+)$, worin $R^+$ Wasserstoff oder Methyl ist, |

bedeuten,

b) ein mit Wasser mischbares, physiologisch unbedenkliches, anionisches oder nichtionisches Tensid oder ein Gemisch mehrerer dieser Tenside und

c) 0 bis 80 Gew.-%, bezogen auf die Zubereitung, eines physiologisch unbedenklichen, wasserlöslichen organischen Lösemittels und

d) 0 bis 70 Gew.-%, bezogen auf die Zubereitung, an Wasser,

welche, bei Anwesenheit von Wasser, ein pH-Wert von weniger als 8 aufweist und welche, nach Verdünnung auf Anwendungskonzentration, als trinkfertige wäßrige Lösung praktisch neutral oder leicht sauer reagiert.

**2.** Wirkstoffzubereitung nach Anspruch 1, gekennzeichnet durch einen Wirkstoff der Formel (I) nach Anspruch 1, in der

$R^1$ Wasserstoff oder $C_1$-$C_4$-Alkyl, vorzugsweise Wasserstoff oder Methyl,

| | |
|---|---|
| $R^2$ | zusammen mit $R^3$ eine Doppelbindung oder |
| $R^2$ und $R^3$ | unabhängig voneinander Wasserstoff oder $C_1$-$C_4$-Alkyl, insbesondere Wasserstoff oder Methyl, |
| X | jeweils unabhängig voneinander Chlor, Brom, $C_1$-$C_4$-Alkyl, vorzugsweise Methyl, Trifluormethyl oder $C_1$-$C_4$-Alkoxy, |
| n | 0, 1 oder 2, |
| Y | Wasserstoff, $C_1$-$C_4$-Halogenoalkyl, insbesondere $C_1$-$C_3$-Alkyl mit ein oder mehreren Fluoratomen als Substituenten, Phenyl oder Phenyl, das durch mindestens einen Rest aus der Gruppe Methyl, Methoxy, Trifluormethyl, Chlor, Methylthio, Methylsulfinyl, Methylsulfonyl, Trifluormethylthio, Trifluormethylsulfinyl oder Trifluormethylsulfonyl substituiert ist, und |
| Z | eine direkte Bindung oder Sauerstoff bedeuten. |

**3.** Wirkstoffzubereitung nach Anspruch 1 oder 2, gekennzeichnet durch einen Wirkstoff der Formel II nach Anspruch 1, in der

| | |
|---|---|
| R und R' | unabhängig voneinander Wasserstoff oder Methyl, |
| W | Sauerstoff, |
| X | jeweils unabhängig voneinander Chlor, $C_1$-$C_4$-Alkoxy, $C_1$-$C_4$-Alkyl, oder Trifluormethyl, |
| n | 0, 1 oder 2, |
| Y | Phenyl oder Phenyl, das durch mindestens einen Substituenten aus der Gruppe Chlor, Trifluormethyl, Trifluormethoxy, Trifluormethylthio, Trifluormethylsulfinyl oder Trifluormethylsulfonyl substituiert ist, und |
| Z | Sauerstoff bedeuten. |

**4.** Wirkstoffzubereitung nach einem oder mehreren der Ansprüche 1 bis 3, gekennzeichnet durch einen Wirkstoff

a) der Formel (Ia)

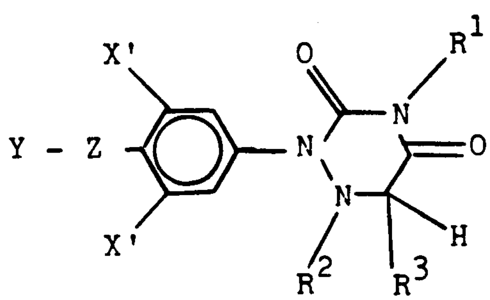

(Ia)

in der

| | |
|---|---|
| $R^1$ | Wasserstoff oder Methyl, |
| $R^2$ | zusammen mit $R^3$ eine Doppelbindung oder |
| $R^2$ und $R^3$ | unabhängig voneinander Wasserstoff oder Methyl, |
| X' | Wasserstoff, Chlor oder Trifluormethyl und |
| Y-Z- | Wasserstoff, $C_1$-$C_4$-Halogenalkoxy, insbesondere Tetrafluorethoxy oder Hexafluorpropoxy, oder $C_1$-$C_4$-Alkylsulfonyl-phenoxy, insbesondere 4-Methylsulfonyl-phenoxy, |

bedeuten.

**5.** Wirkstoffzubereitung nach einem oder mehreren der Ansprüche 1 bis 4, dadurch gekennzeichnet, daß sie aus

a) 100 bis 8000 ppm, bezogen auf die Zubereitung, an einem Wirkstoff der Formel (I) oder (II)

b) einen anionischen oder nichtionischen Tensid mit einem HLB-Wert von mehr als 14,

c) 0 bis 60 Gew.-%, bezogen auf die Zubereitung, eines physiologisch unbedenklichen, wasserlöslichen organischen Lösemittels,

d) 0 bis 10 Gew.-%, bezogen auf die Zubereitung, an Wasser besteht.

**6.** Wirkstoffzubereitung nach einem oder mehreren der Ansprüche 1 bis 5, dadurch gekennzeichnet, daß

das nichtionische Tensid ein Tensid aus der Gruppe der Ether oder Ester von Oligopolyglykolethern mit $C_{12}$-$C_{18}$-Fettsäuren, -Fettalkoholen, -Hydroxyfettsäuren und -estern, Zuckeralkoholen und Zuckerfettsäureestern ist.

**7.** Wirkstoffzubereitung nach Anspruch 6, dadurch gekennzeichnet, daß das Tensid ein Oxethylat eines Sorbitanfettsäureesters ist.

**8.** Verfahren zur Herstellung der nach einem oder mehreren der Ansprüche 1 bis 7 definierten Wirkstoffzubereitungen, dadurch gekennzeichnet, daß man einen oder mehrere Wirkstoffe der obengenannten Formeln (I) und (II) in einem mit Wasser mischbaren, physiologisch unbedenklichen anionischen oder nichtionischen Tensid oder einem Gemisch mehrerer dieser Tenside und gegebenenfalls in Gegenwart von bis zu 80 Gew.-%, bezogen auf die Zubereitung, eines physiologisch unbedenklichen, wasserlöslichen organischen Lösungsmittels und gegebenenfalls in Gegenwart von bis zu 70 Gew.-%, bezogen auf die Zubereitung, an Wasser löst, wobei die entstandene Zubereitung bis zu 10000 ppm Wirkstoffgehalt aufweist.

**9.** Verwendung der nach mindestens einem der Ansprüche 1 bis 7 definierten Wirkstoffzubereitung zur Herstellung von trinkfertigen wäßrigen Lösungen zur Behandlung der Coccidiose bei Tieren.

**10.** Verwendung nach Anspruch 9, dadurch gekennzeichnet, daß die Zubereitung mit Wasser bis zu einem Gehalt von 1 bis 500 ppm Wirkstoff verdünnt wird.

**Patentansprüche für folgenden Vertragsstaat : ES**

**1.** Verfahren zur Herstellung von wasserlöslichen Wirkstoffzubereitungen von Coccidiostatika, dadurch gekennzeichnet, daß man einen oder mehrere Wirkstoffe der allgemeinen Formeln I oder II

(I)

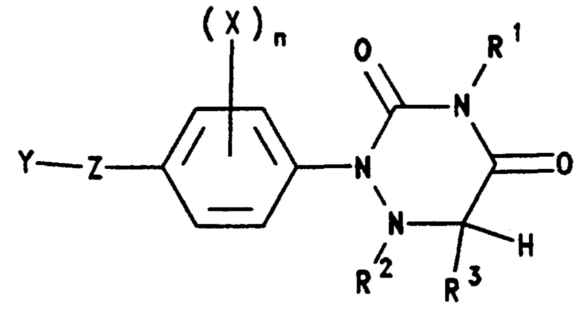

(II)

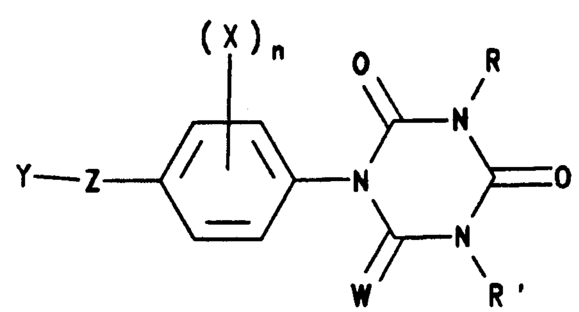

in denen

| | |
|---|---|
| $R^1$, R und R' | unabhängig voneinander Wasserstoff oder $C_1$-$C_4$-Alkyl, |
| $R^2$ | zusammen mit $R^3$ eine Doppelbindung oder unabhängig davon Wasserstoff oder $C_1$-$C_4$-Alkyl, |
| $R^3$ | zusammen mit $R^2$ eine Doppelbindung oder unabhängig davon Wasserstoff oder $C_1$-$C_4$-Alkyl, |
| W | Sauerstoff oder Schwefel, |

X jeweils unabhängig voneinander Halogen, wie Fluor, Chlor und Brom, $C_1$-$C_4$-Alkyl, Trifluormethyl, Cyano, Thiocyanato, $C_1$-$C_4$-Alkylthio, Nitro oder $C_1$-$C_4$-Alkoxy,

n 0, 1, 2, 3 oder 4, vorzugsweise 0 bis 2,

Y Wasserstoff, $C_1$-$C_6$-Alkyl, $C_1$-$C_6$-Halogenalkyl, Benzyl, Phenyl oder Phenyl, das durch mindestens einen Rest aus der Gruppe Alkyl, Alkoxy, Halogenoalkyl, Halogenoalkoxy, Halogen, Alkylthio, Alkylsulfinyl, Alkylsulfonyl, Halogenalkylthio, Halogenalkylsulfinyl und Halogenalkylsulfonyl mit jeweils 1 bis 4 C-Atomen im Alkylrest substituiert ist, Thienyl oder Halothienyl und

Z eine direkte Bindung oder eine divalente Gruppe der Formel O, S, SO, $SO_2$, $NR^0$, worin $R^0$ Wasserstoff oder $C_1$-$C_4$-Alkyl ist, oder $C(CN)(R^*)$, worin $R^*$ Wasserstoff oder Methyl ist,

bedeuten,

in einem mit Wasser mischbaren, physiologisch unbedenklichen, anionischen oder nichtionischen Tensid oder einem Gemisch mehrerer dieser Tenside und gegebenenfalls von bis 80 Gew.-%, bezogen auf die Zubereitung, eines physiologisch unbedenklichen, wasserlöslichen organischen Lösemittels und gegebenenfalls bis zu 70 Gew.-%, bezogen auf die Zubereitung, an Wasser löst, wobei die entstandene Zubereitung bis zu 10000 ppm Wirkstoffgehalt und, bei Anwesenheit von Wasser, ein pH-Wert von weniger als 8 aufweist und diese, nach Verdünnung auf Anwendungskonzentration, als trinkfertige wäßrige Lösung praktisch neutral oder leicht sauer reagiert.

**2.** Verfahren nach Anspruch 1, gekennzeichnet durch einen Wirkstoff der Formel (I) nach Anspruch 1, in der

$R^1$ Wasserstoff oder $C_1$-$C_4$-Alkyl, vorzugsweise Wasserstoff oder Methyl,

$R^2$ zusammen mit $R^3$ eine Doppelbindung oder

$R^2$ und $R^3$ unabhängig voneinander Wasserstoff oder $C_1$-$C_4$-Alkyl, insbesondere Wasserstoff oder Methyl,

X jeweils unabhängig voneinander Chlor, Brom, $C_1$-$C_4$-Alkyl, vorzugsweise Methyl, Trifluormethyl oder $C_1$-$C_4$-Alkoxy,

n 0, 1 oder 2,

Y Wasserstoff, $C_1$-$C_4$-Halogenoalkyl, insbesondere $C_1$-$C_3$-Alkyl mit ein oder mehreren Fluoratomen als Substituenten, Phenyl oder Phenyl, das durch mindestens einen Rest aus der Gruppe Methyl, Methoxy, Trifluormethyl, Chlor, Methylthio, Methylsulfinyl, Methylsulfonyl, Trifluormethylthio, Trifluormethylsulfinyl oder Trifluormethylsulfonyl substituiert ist, und

Z eine direkte Bindung oder Sauerstoff bedeuten.

**3.** Verfahren nach Anspruch 1 oder 2, gekennzeichnet durch einen Wirkstoff der Formel II nach Anspruch 1, in der

R und R' unabhängig voneinander Wasserstoff oder Methyl,

W Sauerstoff,

X jeweils unabhängig voneinander Chlor, $C_1$-$C_4$-Alkoxy, $C_1$-$C_4$-Alkyl, oder Trifluormethyl,

n 0, 1 oder 2,

Y Phenyl oder Phenyl, das durch mindestens einen Substituenten aus der Gruppe Chlor, Trifluormethyl, Trifluormethoxy, Trifluormethylthio, Trifluormethylsulfinyl oder Trifluormethylsulfonyl substituiert ist, und

Z Sauerstoff bedeuten.

**4.** Verfahren nach einem oder mehreren der Ansprüche 1 bis 3, gekennzeichnet durch einen Wirkstoff

a) der Formel (Ia)

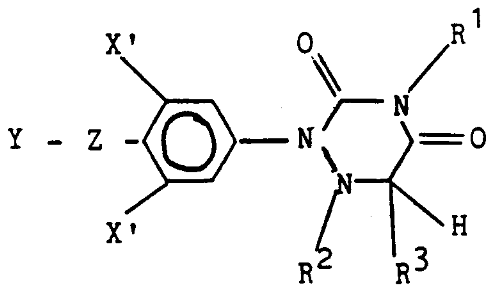

(Ia)

in der

| | |
|---|---|
| $R^1$ | Wasserstoff oder Methyl, |
| $R^2$ | zusammen mit $R^3$ eine Doppelbindung oder |
| $R^2$ und $R^3$ | unabhängig voneinander Wasserstoff oder Methyl, |
| X' | Wasserstoff, Chlor oder Trifluormethyl und |
| Y-Z- | Wasserstoff, $C_1$-$C_4$-Halogenalkoxy, insbesondere Tetrafluorethoxy oder Hexafluorpropoxy, oder $C_1$-$C_4$-Alkylsulfonyl-phenoxy, insbesondere 4-Methylsulfonyl-phenoxy, |

bedeuten.

**5.** Verfahren nach einem oder mehreren der Ansprüche 1 bis 4, dadurch gekennzeichnet, daß die Wirkstoffzubereitung aus
a) 100 bis 8000 ppm, bezogen auf die Zubereitung, an einem Wirkstoff der Formel (I) oder (II)
b) einen anionischen oder nichtionischen Tensid mit einem HLB-Wert von mehr als 14,
c) 0 bis 60 Gew.-%, bezogen auf die Zubereitung, eines physiologisch unbedenklichen, wasserlöslichen organischen Lösemittels,
d) 0 bis 10 Gew.-%, bezogen auf die Zubereitung, an Wasser besteht.

**6.** Verfahren nach einem oder mehreren der Ansprüche 1 bis 5, dadurch gekennzeichnet, daß in der Wirkstoffzubereitung das nichtionische Tensid ein Tensid aus der Gruppe der Ether oder Ester von Oligopolyglykolethern mit $C_{12}$-$C_{18}$-Fettsäuren, -Fettalkoholen, -Hydroxyfettsäuren und -estern, Zuckeralkoholen und Zuckerfettsäureestern ist.

**7.** Verfahren nach Anspruch 6, dadurch gekennzeichnet, daß das Tensid ein Oxethylat eines Sorbitanfettsäureesters ist.

**8.** Verwendung der nach mindestens einem der Ansprüche 1 bis 7 definierten Wirkstoffzubereitung zur Herstellung von trinkfertigen wäßrigen Lösungen zur Behandlung der Coccidiose bei Tieren.

**9.** Verwendung nach Anspruch 8, dadurch gekennzeichnet, daß die Zubereitung mit Wasser bis zu einem Gehalt von 1 bis 500 ppm Wirkstoff verdünnt wird.

## Claims

**Claims for the following Contracting States : AT, BE, CH, DE, FR, GB, IT, LI, NL**

**1.** An active substance preparation which contains
a) up to 10,000 ppm, based on the weight of the preparation, of one or more coccidiostats of the general formulae I or II

(I)

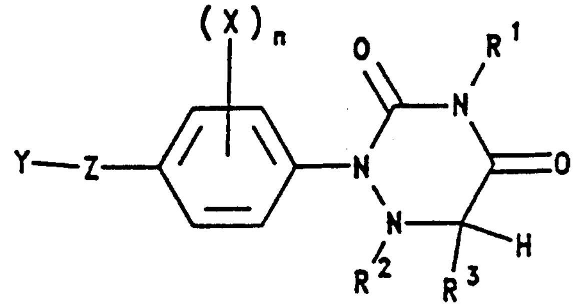

(II)

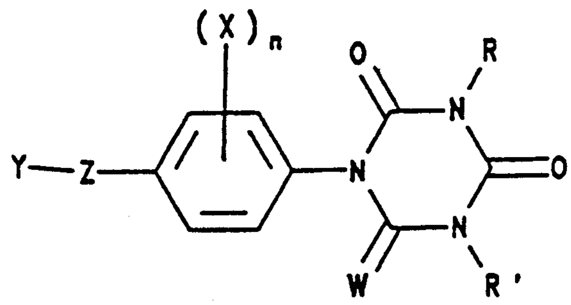

in which

$R^1$, R and R' denote, independently of one another, hydrogen or $C_1$-$C_4$-alkyl,

$R^2$ denotes together with $R^3$ a double bond or independently thereof hydrogen or $C_1$-$C_4$-alkyl,

$R^3$ denotes together with $R^3$ a double bond or independently thereof hydrogen or $C_1$-$C_4$-alkyl,

W denotes oxygen or sulfur,

X denotes, each independently of the others, halogen such as fluorine, chlorine and bromine, $C_1$-$C_4$-alkyl, trifluoromethyl, cyano, thiocyanato, $C_1$-$C_4$-alkylthio, nitro or $C_1$-$C_4$-alkoxy,

n denotes 0, 1, 2, 3 or 4, preferably 0 to 2,

Y denotes hydrogen, $C_1$-$C_6$-alkyl, $C_1$-$C_6$-halogenoalkyl, benzyl, phenyl or phenyl which is substituted by at least one radical from the group comprising alkyl, alkoxy, halogenoalkyl, halogenoalkoxy, halogen, alkylthio, alkylsulfinyl, alkylsulfonyl, halogenoalkylthio, halogenoalkylsulfinyl and halogenoalkylsulfonyl, each alkyl radical having 1 to 4 carbon atoms, or denotes thienyl or halothienyl, and

Z denotes a direct linkage or a divalent group of the formula O, S, SO, $SO_2$, $NR^0$ in which $R^0$ is hydrogen or $C_1$-$C_4$-alkyl, or denotes C(CN)($R^+$) in which $R^+$ is hydrogen or methyl,

b) a water-miscible, physiologically acceptable anionic or nonionic surfactant or a mixture of several of these surfactants, and

c) 0 to 80% by weight, based on the preparation,of a physiologically acceptable water-soluble organic solvent, and

d) 0 to 70% by weight, based on the preparation, of water,

which in the presence of water has a pH below 8 and which after dilution to the use concentration has, as aqueous solution ready for drinking, a virtually neutral or slightly acid reaction.

**2.** An active substance preparation as claimed in claim 1, which contains an active substance of the formula (I) as claimed in claim 1, in which

$R^1$ denotes hydrogen or $C_1$-$C_4$-alkyl, preferably hydrogen or methyl,

$R^2$ denotes together with $R^3$ a double bond, or

$R^2$ and $R^3$ denote, independently of one another, hydrogen or $C_1$-$C_4$-alkyl, especially hydrogen or methyl,

X denotes, each independently of the others, chlorine, bromine, $C_1$-$C_4$-alkyl, preferably

| | |
|---|---|
| | methyl, trifluoromethyl or $C_1$-$C_4$-alkoxy, |
| n | denotes 0, 1 or 2 |
| Y | denotes hydrogen, $C_1$-$C_4$-halogenoalkyl, especially $C_1$-$C_3$-alkyl having one or more fluorine atoms as substituents, phenyl or phenyl which is substituted by at least one radical from the group comprising methyl, methoxy, trifluoromethyl, chlorine, methylthio, methylsulfinyl, methylsulfonyl, trifluoromethylthio, trifluoromethylsulfinyl or trifluoromethylsulfonyl, and |
| Z | denotes a direct linkage or oxygen. |

**3.** An active substance preparation as claimed in claim 1 or 2, which contains an active substance of the formula II as claimed in claim 1, in which

| | |
|---|---|
| R and $R^1$ | denote, independently of one another, hydrogen or methyl, |
| W | denotes oxygen, |
| X | denotes, each independently of the others, chlorine, $C_1$-$C_4$-alkoxy, $C_1$-$C_4$-alkyl or trifluoromethyl, |
| n | denotes 0, 1 or 2, |
| Y | denotes phenyl or phenyl which is substituted by at least one substituent from the group comprising chlorine, trifluoromethyl, trifluoromethoxy, trifluoromethylthio, trifluoromethylsulfinyl or trifluoromethylsulfonyl, and |
| Z | denotes oxygen. |

**4.** An active substance preparation as claimed in one or more of claims 1 to 3, which contains an active substance a) of the formula (Ia)

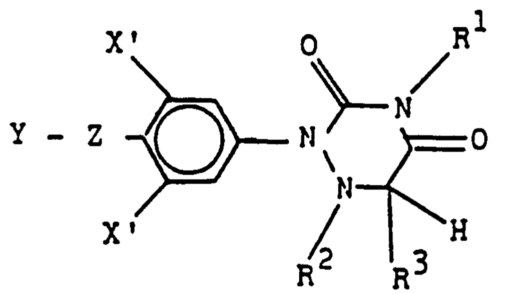

(Ia)

in which

| | |
|---|---|
| $R^1$ | denotes hydrogen or methyl, |
| $R^2$ | denotes together with $R^3$ a double bond, or |
| $R^2$ and $R^3$ | denote, independently of one another, hydrogen or methyl, |
| X' | denotes hydrogen, chlorine or trifluoromethyl, and |
| Y-Z- | denotes hydrogen, $C_1$-$C_4$-halogenoalkoxy, especially tetrafluoroethoxy or hexafluoropropoxy, or $C_1$-$C_4$-alkylsulfonylphenoxy, especially 4-methylsulfonylphenoxy. |

**5.** An active substance preparation as claimed in one or more of claims 1 to 4, which is composed of

a) 100 to 8000 ppm, based on the preparation, of an active substance of the formula (I) or (II),

b) an anionic or nonionic surfactant having an HLB of more than 14,

c) 0 to 60% by weight, based on the preparation, of a physiologically acceptable water-soluble organic solvent,

d) 0 to 10% by weight, based on the preparation, of water.

**6.** An active substance preparation as claimed in one or more of claims 1 to 5, wherein the nonionic surfactant is a surfactant from the group of ethers or esters of oligopolyglycol ethers with $C_{12}$-$C_{18}$ fatty acids, $C_{12}$-$C_{18}$ fatty alcohols, $C_{12}$-$C_{18}$ hydroxy fatty acids and esters, sugar alcohols and sugar fatty acid esters.

**7.** An active substance preparation as claimed in claim 6, wherein the surfactant is an oxyethylate of a sorbitan fatty acid ester.

8. A process for the production of the active substance preparations as defined in one or more of claims 1 to 7, which comprises dissolving one or more active substances of the abovementioned formulae (I) and (II) in a water-miscible, physiologically acceptable anionic or nonionic surfactant, or in a mixture of several of these surfactants, and, where appropriate, in the presence of up to 80% by weight, based on the preparation, of a physiologically acceptable water-soluble organic solvent and, where appropriate, in the presence of up to 70% by weight, based on the preparation, of water, with the resulting preparation containing up to 10,000 ppm active substance.

9. The use of the active substance preparation defined in at least one of claims 1 to 7 for the preparation of aqueous solutions ready for drinking for the treatment of coccidiosis in livestock.

10. The use as claimed in claim 9, wherein the preparation is diluted with water to a content of 1 to 500 ppm active substance.

**Claims for the following Contracting State : ES**

1. A process for the production of water-soluble active substance preparations of coccidiostats, which comprises dissolving one or more active substances of the formulae I or II

(I)

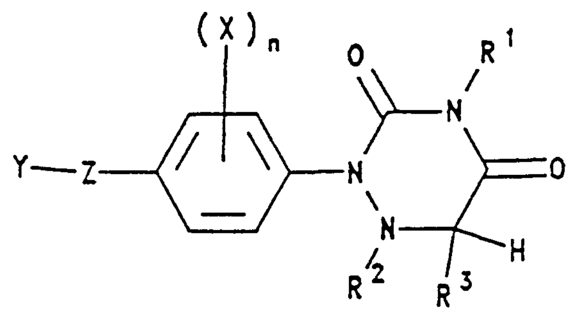

(II)

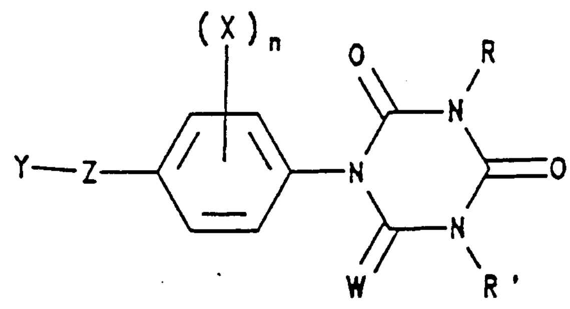

in which

| | |
|---|---|
| $R^1$, R and R' | denote, independently of one another, hydrogen or $C_1$-$C_4$-alkyl, |
| $R^2$ | denotes together with $R^3$ a double bond or independently thereof hydrogen or $C_1$-$C_4$-alkyl, |
| $R^3$ | denotes together with $R^2$ a double bond or independently thereof hydrogen or $C_1$-$C_4$-alkyl, |
| W | denotes oxygen or sulfur, |
| X | denotes, each independently of the others, halogen such as fluorine, chlorine and bromine, $C_1$-$C_4$-alkyl, trifluoromethyl, cyano, thiocyanato, $C_1$-$C_4$-alkylthio, nitro or $C_1$-$C_4$-alkoxy, |
| n | denotes 0, 1, 2, 3 or 4, preferably 0 to 2, |
| Y | denotes hydrogen, $C_1$-$C_6$-alkyl, $C_1$-$C_6$-halogenoalkyl, benzyl, phenyl or phenyl which is substituted by at least one radical from the group comprising alkyl, alkoxy, halogenoalkyl, halogenoalkoxy, halogen, alkylthio, alkylsulfinyl, alkylsulfonyl, halogenoalkylthio, halogenoalkylsulfinyl and halogenoalkylsulfonyl, each alkyl radical |

having 1 to 4 carbon atoms, or denotes thienyl or halothienyl, and

Z denotes a direct linkage or a divalent group of the formula O, S, SO, $SO_2$, $NR^0$ in which $R^0$ is hydrogen or $C_1$-$C_4$-alkyl, or denotes $C(CN)(R^+)$ in which $R^+$ is hydrogen or methyl,

in a water-miscible physiologically acceptable anionic or nonionic surfactant or in a mixture of several of these surfactants, and, where appropriate, in the presence of up to 80% by weight, based on the preparation, of a physiologically acceptable water-soluble organic solvent and, where appropriate, in the presence of up to 70% by weight, based on the preparation, of water, with the resulting preparation containing up to 10,000 ppm active substance and in the presence of water having a pH below 8, and the latter having after dilution to the use concentration, as aqueous solution ready for drinking, a virtually neutral or slightly acid reaction.

**2.** The process as claimed in claim 1, wherein the active substance has the formula (I) as claimed in claim 1, in which

$R^1$ denotes hydrogen or $C_1$-$C_4$-alkyl, preferably hydrogen or methyl,

$R^2$ denotes together with $R^3$ a double bond, or

$R^2$ and $R^3$ denote, independently of one another, hydrogen or $C_1$-$C_4$-alkyl, especially hydrogen or methyl,

X denotes, each independently of the others, chlorine, bromine, $C_1$-$C_4$-alkyl, preferably methyl, trifluoromethyl or $C_1$-$C_4$-alkoxy,

n denotes 0, 1 or 2

Y denotes hydrogen, $C_1$-$C_4$-halogenoalkyl, especially $C_1$-$C_3$-alkyl having one or more fluorine atoms as substituents, phenyl or phenyl which is substituted by at least one radical from the group comprising methyl, methoxy, trifluoromethyl, chlorine, methylthio, methylsulfinyl, methylsulfonyl, trifluoromethylthio, trifluoromethylsulfinyl or trifluoromethylsulfonyl, and

Z denotes a direct linkage or oxygen.

**3.** The process as claimed in claim 1 or 2, wherein the active substance has the formula II as claimed in claim 1, in which

R and R' denote, independently of one another, hydrogen or methyl,

W denotes oxygen,

X denotes, each independently of the others, chlorine, $C_1$-$C_4$-alkoxy, $C_1$-$C_4$-alkyl or trifluoromethyl,

n denotes 0, 1 or 2,

Y denotes phenyl or phenyl which is substituted by at least one substituent from the group comprising chlorine, trifluoromethyl, trifluoromethoxy, trifluoromethylthio, trifluoromethylsulfinyl or trifluoromethylsulfonyl, and

Z denotes oxygen.

**4.** The process as claimed in one or more of claims 1 to 3, wherein the active substance a) has the formula (Ia)

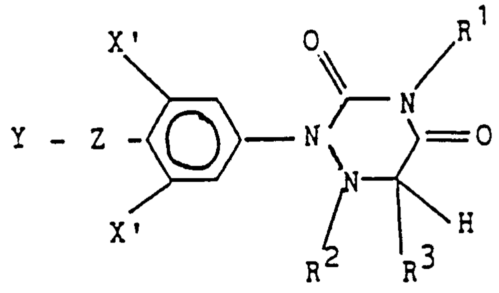

(Ia)

in which

$R^1$ denotes hydrogen or methyl,

$R^2$ denotes together with $R^3$ a double bond, or

$R^2$ and $R^3$ denote, independently of one another, hydrogen or methyl,

X' denotes hydrogen, chlorine or trifluoromethyl, and

Y-Z- denotes hydrogen, $C_1$-$C_4$-halogenoalkoxy, especially tetrafluoroethoxy or hexafluoropropoxy, or $C_1$-$C_4$-alkylsulfonylphenoxy, especially 4-methylsulfonylphenoxy.

**5.** The process as claimed in one or more of claims 1 to 4, wherein the active substance preparation is composed of

a) 100 to 8000 ppm, based on the preparation, of an active substance of the formula (I) or (II),

b) an anionic or nonionic surfactant having an HLB of more than 14,

c) 0 to 60% by weight, based on the preparation, of a physiologically acceptable water-soluble organic solvent,

d) 0 to 10% by weight, based on the preparation, of water.

**6.** The process as claimed in one or more of claims 1 to 5, wherein the nonionic surfactant in the active substance preparation is a surfactant from the group of ethers or esters of oligopolyglycol ethers with $C_{12}$-$C_{18}$ fatty acids, $C_{12}$-$C_{18}$ fatty alcohols, $C_{12}$-$C_{18}$ hydroxy fatty acids and esters, sugar alcohols and sugar fatty acid esters.

**7.** The process as claimed in claim 6, wherein the surfactant is an oxyethylate of a sorbitan fatty acid ester.

**8.** The use of the active substance preparation defined in at least one of claims 1 to 7 for the preparation of aqueous solutions ready for drinking for the treatment of coccidiosis in livestock.

**9.** The use as claimed in claim 8, wherein the preparation is diluted with water to a content of 1 to 500 ppm active substance.

**Revendications**

**Revendications pour les Etats contractants suivants : AT, BE, CH, DE, FR, GB, IT, LI, NL**

**1.** Préparation de principe actif, caractérisée en ce qu'elle contient

a) jusqu'à 10 000 ppm, par rapport au poids de la préparation, d'un ou plusieurs coccidiostatiques de formules générales (I) ou (II)

(I)

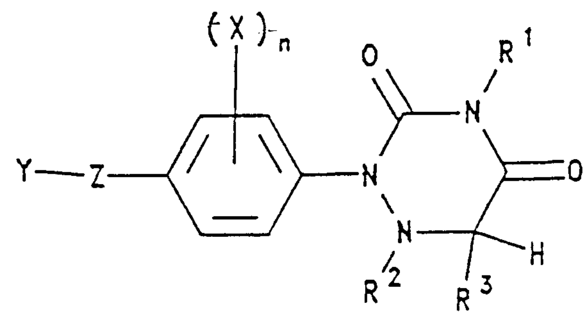

(II)

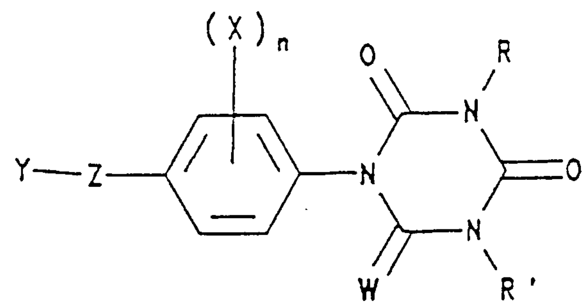

dans lesquelles

$R^1$, R et R', indépendamment les uns des autres, représentent chacun un hydrogène ou un radical alkyle en $C_1$-$C_4$,

$R^2$ forme avec $R^3$ une double liaison ou, indépendamment de ce dernier, représente un hydrogène ou un radical alkyle en $C_1$-$C_4$,

$R^3$ forme avec $R^2$ une double liaison ou, indépendamment de ce dernier, représente un hydrogène ou un radical alkyle en $C_1$-$C_4$,

W est un oxygène ou un soufre,

chacun des radicaux X, indépendamment des autres, est un halogène, comme par exemple le fluor, le chlore ou le brome, ou un radical alkyle en $C_1$-$C_4$, trifluorométhyle, cyano, thiocyanato, alkylthio en $C_1$-$C_4$, nitro ou alcoxy en $C_1$-$C_4$,

n vaut 0, 1, 2, 3 ou 4, de préférence de 0 à 2,

Y est un hydrogène ou un radical alkyle en $C_1$-$C_6$, halogénoalkyle en $C_1$-$C_6$, benzyle, phényle, ou encore phényle substitué par au moins un radical choisi parmi l'ensemble comprenant les groupes alkyle, alcoxy, halogénoalkyle, halogénoalcoxy, halogéno, alkylthio, alkylsulfinyle, alkylsulfonyle, halogénoalkylthio, halogénoalkylsulfinyle et halogénoalkylsulfonyle, avec chaque fois 1 à 4 atomes de carbone dans le radical alkyle, thiényle ou halogénothiényle, et

Z représente une liaison directe ou un groupe divalent de formule O, S, SO, $SO_2$, NH, $NR^0$, où $R^0$ représente un hydrogène ou un radical alkyle en $C_1$-$C_4$, ou encore C(CN)($R^+$), où $R^+$ est un hydrogène ou un radical méthyle,

b) un tensioactif miscible à l'eau, physiologiquement non toxique, anionique ou non ionique, ou un mélange de plusieurs de ces tensioactifs, et

c) 0 à 80 % en poids, par rapport à la préparation, d'un solvant organique hydrosoluble physiologiquement inoffensif,

d) 0 à 70 % en poids d'eau par rapport à la préparation,

qui, en présence d'eau, présente un pH inférieur à 8, et qui, après dilution à la concentration d'utilisation, réagit d'une manière pratiquement neutre ou faiblement acide à une solution aqueuse prête à boire.

**2.** Préparation de principe actif selon la revendication 1, caractérisée en ce qu'elle contient un principe actif de formule (I) selon la revendication 1, dans laquelle

$R^1$ est un hydrogène ou un radical alkyle en $C_1$-$C_4$, de préférence un hydrogène ou le radical méthyle,

$R^2$ forme avec $R^3$ une double liaison, ou bien $R^2$ et $R^3$, indépendamment l'un de l'autre, sont chacun un hydrogène ou un radical alkyle en $C_1$-$C_4$, en particulier un hydrogène ou le radical méthyle,

les radicaux X, indépendamment les uns des autres, représentent chacun le chlore, le brome ou un radical alkyle en $C_1$-$C_4$, de préférence méthyle, trifluorométhyle, ou encore alcoxy en $C_1$-$C_4$,

n vaut 0, 1 ou 2,

Y est un hydrogène ou un radical halogénoalkyle en $C_1$-$C_4$, en particulier un radical alkyle en $C_1$-$C_3$ comportant un ou plusieurs atomes de fluor servant de substituants, phényle, ou encore phényle substitué par au moins un radical appartenant à l'ensemble comprenant les groupes méthyle, méthoxy, trifluorométhyle, chloro, méthylthio, méthylsulfinyle, méthylsulfonyle, trifluorométhylthio, trifluorométhylsulfinyle ou trifluorométhylsulfonyle, et

Z est une liaison directe ou un oxygène.

**3.** Préparation de principe actif selon la revendication 1 ou 2, caractérisée en ce qu'elle contient un principe actif de formule (II) selon la revendication 1, dans laquelle

R et R', indépendamment l'un de l'autre, représentent chacun un hydrogène ou le radical méthyle,

W est un oxygène,

les radicaux X, indépendamment les uns des autres, représentent chacun un radical chloro, alcoxy en $C_1$-$C_4$, alkyle en $C_1$-$C_4$ ou trifluorométhyle,

n vaut 0, 1 ou 2,

Y est le radical phényle ou un radical phényle substitué par au moins un substituant appartenant au groupe comprenant les radicaux chloro, trifluorométhyle, trifluorométhoxy, trifluorométhylthio, trifluorométhylsulfinyle ou trifluorométhylsulfonyle, et

Z est un oxygène.

**4.** Préparation de principe actif selon l'une ou plusieurs des revendications 1 à 3, caractérisée en ce qu'elle contient un principe actif a) de formule (Ia)

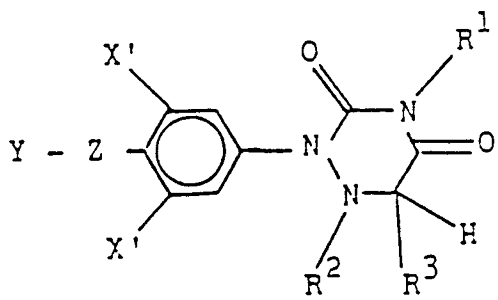

dans laquelle
$R^1$ est un hydrogène ou le radical méthyle,
$R^2$ forme avec $R^3$ une double liaison, ou encore $R^2$ et $R^3$, indépendamment l'un de l'autre, représentent chacun un hydrogène ou le radical méthyle,
X' est un hydrogène ou le radical chloro ou trifluorométhyle, et
Y-Z représente un hydrogène ou un radical halogénoalcoxy en $C_1$-$C_4$, en particulier tétrafluoréthoxy ou hexafluoropropoxy, ou encore un radical (alkyle en $C_1$-$C_4$)sulfonylphénoxy,en particulier méthylsulfonyl-4 phénoxy.

**5.** Préparation de principe actif selon l'une ou plusieurs des revendications 1 à 4, caractérisée en ce qu'elle comprend :
a) 100 à 8000 ppm, par rapport à la préparation, d'un principe actif de formule (I) ou (II),
b) un tensioactif anionique ou non ionique ayant un rapport hydro-lipophile supérieur à 14,
c) 0 à 60 % en poids, par rapport à la préparation, d'un solvant organique hydrosoluble physiologiquement inoffensif,
d) 0 à 10 % en poids d'eau par rapport à la préparation.

**6.** Préparation de principe actif selon l'une ou plusieurs des revendications 1 à 5, caractérisée en ce que le tensioactif non ionique est un tensioactif du groupe des éthers ou esters de produits d'addition oligo-oxyéthylénés et d'acides gras, d'alcools gras, d'hydroxyacides gras et d'esters gras en $C_{12}$-$C_{18}$, d'alcools de sucre et d'esters d'acides gras du sucre.

**7.** Préparation de principe actif selon la revendication 6, caractérisée en ce que le tensioactif est un produit d'éthoxylation d'un ester d'acide gras du sorbitol.

**8.** Procédé pour préparer les préparations de principe actif selon l'une ou plusieurs des revendications 1 à 7, caractérisé en ce qu'on dissout un ou plusieurs principes actifs ayant les formules (I) et (II) ci-dessus dans un tensioactif anionique ou non ionique, physiologiquement inoffensif, miscible à l'eau, ou dans un mélange de plusieurs de ces tensioactifs, et éventuellement en présence au plus de 80 % en poids, par rapport à la préparation, d'un solvant organique hydrosoluble physiologiquement inoffensif, et éventuellement en présence au plus de 70 % en poids d'eau par rapport à la préparation, la préparation obtenue ayant une teneur en principe actif inférieur ou égale à 10 000 ppm.

**9.** Utilisation de la préparation de principe actif selon l'une des revendications 1 à 7, pour préparer des solutions aqueuses prêtes à boire servant au traitement de la coccidiose chez l'animal.

**10.** Utilisation selon la revendication 9, caractérisée en ce que la préparation est diluée à l'eau jusqu'à une teneur en principe actif de 1 à 500 ppm.

**Revendications pour l'Etat contractant suivant : ES**

**1.** Procédé pour obtenir des préparations hydrosolubles de principe actif de coccidiostatiques, caractérisé en ce qu'on dissout un ou plusieurs principes actifs ayant les formules (I) et (II)

(I)

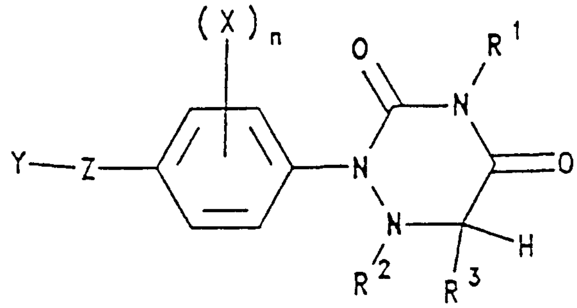

(II)

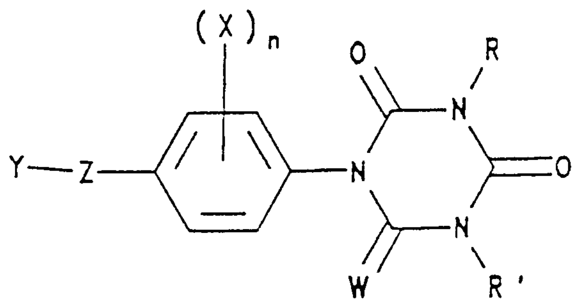

dans lesquelles

$R^1$, R et R', indépendamment les uns des autres, représentent chacun un hydrogène ou un radical alkyle en $C_1$-$C_4$,

$R^2$ forme avec $R^3$ une double liaison ou, indépendamment de ce dernier, représente un hydrogène ou un radical alkyle en $C_1$-$C_4$,

$R^3$ forme avec $R^2$ une double liaison ou, indépendamment de ce dernier, représente un hydrogène ou un radical alkyle en $C_1$-$C_4$,

W est un oxygène ou un soufre,

chacun des radicaux X, indépendamment des autres, est un halogène, comme par exemple le fluor, le chlore ou le brome, ou un radical alkyle en $C_1$-$C_4$, trifluorométhyle, cyano, thiocyanato, alkylthio en $C_1$-$C_4$, nitro ou alcoxy en $C_1$-$C_4$,

n vaut 0, 1, 2, 3 ou 4, de préférence de 0 à 2,

Y est un hydrogène ou un radical alkyle en $C_1$-$C_6$, halogénoalkyle en $C_1$-$C_6$, benzyle, phényle, ou encore phényle substitué par au moins un radical choisi parmi l'ensemble comprenant les groupes alkyle, alcoxy, halogénoalkyle,halogénoalcoxy, halogéno, alkylthio, alkylsulfinyle, alkylsulfonyle, halogénoalkylthio, halogénoalkylsulfinyle et halogénoalkylsulfonyle, avec chaque fois 1 à 4 atomes de carbone dans le radical alkyle, thiényle ou halogénothiényle, et

Z représente une liaison directe ou un groupe divalent de formule O, S, SO, $SO_2$, NH, $NR^0$, où $R^0$ représente un hydrogène ou un radical alkyle en $C_1$-$C_4$, ou encore C(CN)($R^+$), où $R^+$ est un hydrogène ou un radical méthyle,

dans un tensioactif anionique ou non ionique, physiologiquement inoffensif, miscible à l'eau, ou dans un mélange de plusieurs de ces tensioactifs, et éventuellement en présence au plus de 80 % en poids, par rapport à la préparation, d'un solvant organique hydrosoluble physiologiquement inoffensif, et éventuellement en présence au plus de 70 % en poids d'eau par rapport à la préparation, la préparation obtenue ayant une teneur en principe actif inférieur ou égale à 10 000 ppm, puis, après dilution à la concentration d'utilisation, on les fait réagir, en tant que solution aqueuse prête à l'emploi, selon une réaction pratiquement neutre ou faiblement acide.

**2.** Procédé selon la revendication 1, caractérisé en ce qu'il contient un principe actif de formule (I) selon la revendication 1, dans laquelle

$R^1$ est un hydrogène ou un radical alkyle en $C_1$-$C_4$, de préférence un hydrogène ou le radical méthyle,

$R^2$ forme avec $R^3$ une double liaison, ou bien $R^2$ et $R^3$, indépendamment l'un de l'autre, sont chacun un hydrogène ou un radical alkyle en $C_1$-$C_4$, en particulier un hydrogène ou le radical méthyle,

les radicaux X, indépendamment les uns des autres, représentent chacun le chlore, le brome ou un radical alkyle en $C_1$-$C_4$, de préférence méthyle, trifluorométhyle, ou encore alcoxy en $C_1$-$C_4$,

n vaut 0, 1 ou 2,

Y est un hydrogène ou un radical halogénoalkyle en $C_1$-$C_4$, en particulier un radical alkyle en $C_1$-$C_3$ comportant un ou plusieurs atomes de fluor servant de substituants, phényle, ou encore phényle substitué par au moins un radical appartenant à l'ensemble comprenant les groupes méthyle, méthoxy, trifluorométhyle, chloro, méthylthio, méthylsulfinyle, méthylsulfonyle, trifluorométhylthio, trifluorométhylsulfinyle ou trifluorométhylsulfonyle, et

Z est une liaison directe ou un oxygène.

**3.** Procédé selon la revendication 1 ou 2, caractérisé en ce qu'il contient un principe actif de formule (II) selon la revendication 1, dans laquelle

R et R', indépendamment l'un de l'autre, représentent chacun un hydrogène ou le radical méthyle,

W est un oxygène,

les radicaux X, indépendamment les uns des autres, représentent chacun un radical chloro, alcoxy en $C_1$-$C_4$, alkyle en $C_1$-$C_4$ ou trifluorométhyle,

n vaut 0, 1 ou 2,

Y est le radical phényle ou un radical phényle substitué par au moins un substituant appartenant au groupe comprenant les radicaux chloro, trifluorométhyle, trifluorométhoxy, trifluorométhylthio, trifluorométhylsulfinyle ou trifluorométhylsulfonyle, et

Z est un oxygène.

**4.** Procédé selon l'une ou plusieurs des revendications 1 à 3, caractérisé en ce qu'il contient un principe actif

a) de formule (Ia)

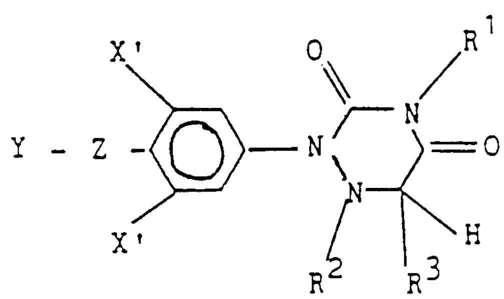

(Ia)

dans laquelle

$R^1$ est un hydrogène ou le radical méthyle,

$R^2$ forme avec $R^3$ une double liaison, ou encore $R^2$ et $R^3$, indépendamment l'un de l'autre, représentent chacun un hydrogène ou le radical méthyle,

X' est un hydrogène ou le radical chloro ou trifluorométhyle, et

Y-Z représente un hydrogène ou un radical halogénoalcoxy en $C_1$-$C_4$, en particulier tétrafluoréthoxy ou hexafluoropropoxy, ou encore un radical (alkyle en $C_1$-$C_4$)sulfonylphénoxy,en particulier méthylsulfonyl-4 phénoxy.

**5.** Procédé selon l'une ou plusieurs des revendications 1 à 4, caractérisé en ce que la préparation de principe actif comprend

a) 100 à 8000 ppm, par rapport à la préparation, d'un principe actif de formule (I) ou (II),

b) un tensioactif anionique ou non ionique ayant un rapport hydro-lipophile supérieur à 14,

c) 0 à 60 % en poids, par rapport à la préparation, d'un solvant organique hydrosoluble physiologiquement inoffensif,

d) 0 à 10 % en poids d'eau par rapport à la préparation.

**6.** Procédé selon l'une ou plusieurs des revendications 1 à 5, caractérisé en ce que, dans la préparation de tensioactifs, le tensioactif non ionique est un tensioactif du groupe des éthers ou esters de produits d'addition oligooxyéthylénés et d'acides gras, d'alcools gras, d'hydroxyacides gras et d'esters gras en

$C_{12}$-$C_{18}$, d'alcools de sucre et d'esters d'acides gras du sucre.

**7.** Procédé selon la revendication 6, caractérisé en ce que le tensioactif est un produit d'éthoxylation d'un ester d'acide gras du sorbitol.

**8.** Utilisation de la préparation de principe actif selon au moins l'une des revendications 1 à 7, pour préparer des solutions aqueuses prêtes à boire servant au traitement de la coccidiose chez l'animal.

**9.** Utilisation selon la revendication 8, caractérisée en ce que la préparation est diluée à l'eau jusqu'à une teneur en principe actif de 1 à 500 ppm.